# EUROPEAN PATENT APPLICATION

(11) **EP 3 919 089 A1**
(43) Date of publication of application: **08.12.2021**
(21) Application number: 20178152.3
(22) Date of filing: 04.06.2020
(51) Int. Cl.: A61L 9/20, A61L 2/10, C02F 1/00, C02F 1/32, F24F 3/16

(54) **A STERILIZATION SYSTEM HAVING AN LED UV EMITTER AND POROUS SCATTERING MEDIUM**

(71) Applicant: Lumileds LLC, San Jose, CA 95131 (US)
(72) Inventor: The designation of the inventor has not yet been filed
(74) Representative: dompatent von Kreisler Selting Werner - Partnerschaft von Patent- und Rechtsanwälten mbB

(57) **Abstract**

A sterilization system includes a semiconductor LED emitter of UV radiation. A fluid flow chamber is positioned to receive UV radiation from one or more UV emitters. A porous UV scattering medium positioned within the fluid flow chamber to scatter UV radiation while allowing fluid flow therethrough. In one embodiment the pores are relatively large, being sized to have pores that do not directly filter microorganisms.

## Description

### TECHNICAL FIELD

The present disclosure generally relates to a portable or low power fluid sterilization or germicidal system that uses ultraviolet radiation. In one embodiment, a sterilization system includes a porous structure with a high ultraviolet radiation scattering to ensure microorganism contact with UV radiation.

### BACKGROUND

Semiconductor light-emitting devices (LEDs) can be manufactured to emit high intensity ultraviolet germicidal radiation. Ultraviolet germicidal irradiation (UVGI) is a disinfection method that uses short-wavelength ultraviolet (UV-c) light to kill or inactivate microorganisms by destroying nucleic acids and disrupting their DNA, leaving them unable to perform vital cellular functions. UVGI is used in a variety of applications, such as food, air, and water purification. The application of UVGI to disinfection has been an accepted practice since the mid-20th century. While it has long been used in medical sanitation and sterile work facilities, UV radiation has increasingly been employed to sterilize drinking and wastewater. Contaminated fluid can be are enclosed and recirculated to ensure a higher exposure to the UV radiation.

Current UVGI systems are designed to expose environments such as water tanks, sealed rooms and forced air systems to germicidal UV. Exposure comes from germicidal lamps that emit germicidal UV at the correct wavelength, thus irradiating the environment. The forced flow of air or water through this environment ensures exposure. These systems generally use mercury-based lamps operating at low vapor pressure, pulsed-xenon lamps, or more recently, ultraviolet light-emitting diodes (UV-C LED) that emit light at wavelengths between 230 nm and 280 nm.

UV-C LEDs offer many advantages over traditional UV-emitting lamps. The reduced size of LEDs opens up options for small reactor systems allowing for point-of-use applications and integration into medical devices. The low power consumption of LEDs also lends itself to portable operation, and the "instant on" features of LEDs allow these sources to achieve a longer installed time than a traditional germicidal lamp in intermittent use.

One drawback of LED based UVGI systems is the lower power output. The effectiveness of germicidal UV depends on the length of time a microorganism is exposed to UV, the intensity and wavelength of the UV radiation, the presence of particles that can protect the microorganisms from UV, and microorganism ability to withstand UV exposure. In many systems, ensuring that microorganisms are exposed to sterilizing levels of UV requires multiple passes of a fluid through a UV radiation zone. This is necessary so that the UV is effective against the highest number of microorganisms and irradiates resistant microorganisms more than once to break them down. However, this is difficult with power efficient LED-based systems with relatively low UV radiation intensity that need long exposure time of the contamination fluid in order to achieve the same sterilization efficacy as high power mercury-based UV lamps.

Unfortunately, many applications support only limited circulation space, require low speed fluid flow, and are not able to provide repeated recirculation of fluid. What is needed for portable sterilizing applications are single pass LED UV systems that provide adequate sterilization without a need for fluid recirculation or long retention times in a UV sterilization zone or chamber. To improve usefulness, such systems can be battery operated or low power for portable applications.

### SUMMARY

In accordance with embodiments of the invention, a sterilization system includes a semiconductor LED emitter of UV radiation and a fluid flow chamber positioned to receive UV radiation. A porous UV scattering medium is positioned within the fluid flow chamber to scatter UV radiation while allowing fluid flow therethrough.

In some embodiments, the UV radiation is UV-C radiation.

In some embodiments, the fluid flow chamber further comprises a UV radiation opaque housing.

In some embodiments, the fluid flow chamber further comprises a UV radiation reflective material.

In some embodiments, the fluid flow chamber is positioned to receive UV radiation from multiple semiconductor LED emitters.

In some embodiments, the porous UV scattering medium further comprises porous quartz.

In some embodiments, the porous UV scattering medium is sized to have pores that do not directly filter microorganisms.

In some embodiments, the semiconductor LED emitter is battery powered.

In some embodiments, the semiconductor LED emitter is powered by a low voltage electrical system.

In some embodiments, the sterilization system is included in a portable system.

In some embodiments, the sterilization system is included in a portable mask.

In some embodiments, the sterilization system is included in an automotive air filtering system.

In some embodiments, the sterilization system further comprises an air filtering system.

In some embodiments, an LED control system and a connected sensor is included, with semiconductor LED emitter operation varying according to sensed data received from the connected sensor.

In some embodiments, the fluid flow chamber is connected to an air system.

In some embodiments, the fluid flow chamber is connected to a liquid system.

In another embodiment, a sterilization system includes a semiconductor LED emitter of UV radiation and a fluid flow chamber positioned to receive UV radiation. A porous UV scattering medium is positioned within the fluid flow chamber to scatter UV radiation while allowing fluid flow therethrough. An LED controller is connected to a sensor system that monitors fluid flow through the fluid flow chamber, with the semiconductor LED emitter of UV radiation intensity controlled based at least in part on fluid flow conditions.

In some embodiments, the semiconductor LED emitter of UV radiation emits sterilizing levels of UV radiation when fluid flows in a first direction and does not emit sterilizing levels of radiation when fluid flows in a second direction.

In some embodiments, the sterilization system further comprises a battery powered mask.

In another embodiment, a sterilization method includes emitting sterilizing UV radiation with a semiconductor LED emitter. A fluid flow chamber is positioned to receive sterilizing UV radiation and a porous UV scattering medium positioned within the fluid flow chamber and allowed to scatter UV radiation while allowing fluid flow therethrough.

### BRIEF DESCRIPTION OF THE DRAWINGS

Non-limiting and non-exhaustive embodiments of the present disclosure are described with reference to the following figures, wherein like reference numerals refer to like parts throughout the various figures unless otherwise specified.
FIG. 1 is an illustration a UV sterilization system;
FIG. 2 is an illustration of a UV sterilization system with optional pre- and post-filters;
FIG. 3 is an illustration of a low power, small form factor air purification system supporting UV sterilization; and
FIG. 4 is an illustration of a battery powered face mask supporting UV sterilization.

### DETAILED DESCRIPTION

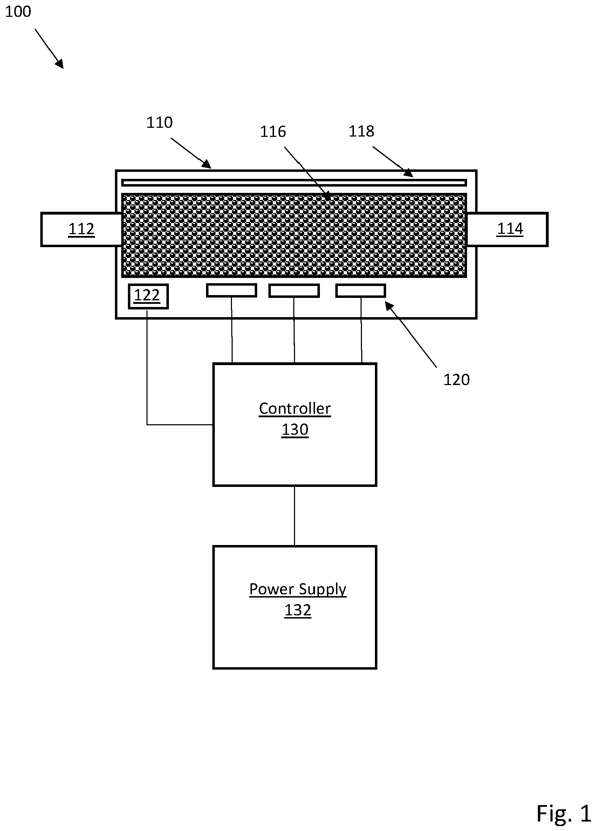

FIG. 1 is an illustration a UV sterilization system 100 that includes a housing 110 having a fluid inlet 112, a fluid outlet 114, and an optional UV reflector 118. Contained within the housing 110 is a porous material 116 that allows fluid flow therethrough from the inlet 112 to the outlet 114. Also positioned within or near the housing are one or more LED UV emitters 120 able to direct UV radiation into the porous material 116. UV light intensity or fluid flow characteristics can be monitored with an optional sensor system 122. Operation of the LED UV emitters 120 and sensor system 122 can be controlled by controller 130. A power supply 132 such as a battery or low voltage (e.g. 12 volt) electrical system can be used to power UV sterilization system 100.

In operation, fluid that is contaminated with microorganisms such as viruses, bacteria, yeasts, molds, or otherwise in need of UV treatment can travel from inlet 112 to outlet 114 through the porous material 116. The porous material 116 provides a scattering zone for UV radiation emitted by the one or more LED UV emitters 120. In effect, for air or gasses, the porous material 116 acts as a coarse filter for the fluid, causing small vortexes of turbulence to decrease the effective flow rate of the air, and also acts as a scattering material for the UV light to increase its effective path length through the fluid. For liquid fluids, turbulent flow is generally not available, but the porous material 116 can still act to scatter UV radiation. In typical applications, scattering provided by the porous material 116 can increase the UV light pathlength by 10 or 100 times (or more). In those embodiments with an included UV reflector 118, UV light pathlength can be even greater due to one or more reflections of UV radiation back into the porous material 116. In some embodiments, scattering materials can be arranged to promote directional scattering or otherwise provide a scattering gradient or pattern. For example, in some embodiments scattering material can be arranged to encourage a scattering pattern along a fluid flow direction, while in other embodiments scattering patterns can locally increase or decrease exposure of fluid to UV radiation. Such embodiments allow, for example, high intensity UV radiation treatment of fast moving fluid zones, while slow moving fluid zones can be treated for a longer duration with a reduced UV radiation intensity.

In some embodiments the housing 110 provides complete or partial UV blocking. The housing 110 can be constructed with a UV opaque material. Alternatively, or in addition, UV reflectors or absorbers can be attached or applied to inner or outer walls of the housing 110 to block UV radiation. In some embodiments, the housing 110 can directly support one or more attached LED UV emitters 120, while in other embodiments a window or gap in the housing can be made to allow UV radiation from separately mounted LED UV emitters to be directed at the porous material. Similarly, the optional sensor system 122 can be mounted within the housing 110, mounted on an outside of the housing 110, or be separately mounted from the housing 110 with access via a window or gap in the housing. While the housing 110 can be of any shape and size able to support or contain fluid flow through the porous material 116 at needed fluid flow rates, often the housing 110 will have longer dimensions in a direction of fluid flow. Also, in many embodiments, the housing and contained porous material will have a volume less than 100 cubic centimeters, although larger volumes are possible for certain applications and available power.

In some embodiments, the inlets 112 and 114 can include multiple inlets and outlets or be connected to respective fluid manifolds (not shown). Size of inlets and outlets can vary, with some embodiments having inlets/outlets that are smaller than the housing 110, while in other embodiments they are sized to be about the same size as the housing 110. In some embodiments, the inlet 112 can be attached to process biological fluids passing from medical devices such as tubing, catheters, syringes, or other medical, pharmaceutical, or therapeutic fluids. In other embodiments, air or other gasses can be blown or moved by pressure differentials between the inlet 112 and outlet 114. In some embodiments, fluid flow movement can be reversed, with fluid moving from the outlet 114 toward the inlet 112.

In some embodiments the porous material 116 can formed from porous quartz, fused silica, or sapphire having an porous structure. In other embodiments, ultraviolet transparent fluoropolymers can be used. Pore or fluid channel sizes in the porous material 116 are much larger than is typically needed to filter sub-micron sized microorganisms and will typically be on the order of 100 microns to one millimeter in size. As will be understood, the porous material can include a wide range of regularly or irregularly arranged fluid flow capable scattering structures. The porous material can include but is not limited to manufacture from material such as regularly or irregularly arranged spheres, frits, lattice structure, sponge-like structures, or other macroscopic open pore structures. The porous material can be provided as a rigid slab or block with pores extending therethrough, or in some embodiments can be formed from packed particles or structures held together by the housing 110. In some embodiments, porous materials can include fluid flow capable scattering structures such as flow tubes, bundles of micro pipes, capillary tubes, or other channel systems. In some embodiments, channels of a porous material can include turbulence inducing structures attached to pore channel walls or positioned within a pore channel. In other embodiments, multiple types of porous materials can be used, with for example, an array of flow tubes alternated with a porous frit structure.

In some embodiments the one or more LED UV emitters 120 can be constructed from patterned or unpatterned sapphire, silicon, or silicon carbide that is able to support an epitaxially grown or deposited semiconductor LED layers. In one embodiment, a semiconductor p-layer can be sequentially grown or deposited on an n-layer, forming an active region at the junction between layers. Semiconductor materials capable of forming high-brightness light emitting devices can include, but are not limited to, Group III-V semiconductors, particularly binary, ternary, and quaternary alloys of gallium, aluminum, indium, and nitrogen, also referred to as III-nitride materials. UV wavelength emitted can range from 230nm to 310nm, with narrower ranges possible. In some embodiments, several LED UV emitters having differing UV wavelength emission characteristics can be used. Advantageously, use of multiple UV wavelengths can allow better targeting of, for example, bacterial versus viral microorganisms.

The LED UV emitters 120 can be packaged and hermetically sealed against fluid using UV tolerant materials such as spin on glass. In some embodiments, the LED UV emitters 120 be attached to a submount or printed circuit board also supporting controller 130 that is connected for powering and controlling UV emission by the semiconductor LED. In certain embodiments, the printed circuit board can also include electrical vias, heat sinks, ground planes, electrical traces, and flip chip or other mounting systems. The submount or printed circuit board may be formed of any suitable material, such as ceramic, silicon, aluminum, etc. If the submount material is conductive, an insulating layer is formed over the substrate material, and the metal electrode pattern is formed over the insulating layer. The submount acts as a mechanical support, provides an electrical interface between electrodes on the LED and a power supply, and provides heat sinking.

In still other embodiments primary or secondary optics can be attached or positioned near LED UV emitters 120. Optics can include wave guides, concave or convex lenses, lenslet arrays, graded index lens, reflectors, scattering elements, beam homogenizers, diffusers, or other light focusing or blurring optics. Protective layers, transparent layers, thermal layers, or other packaging structures can be used as needed for specific applications.

In some embodiments the sensor system 112 can be used to monitor UV radiation output. Wavelength ranges and intensity can be measured, with adjustments to power supplied to the LED UV emitters 120 being made as necessary by the controller 130. In other embodiments, the sensor system 112 can include fluid flow detectors that monitor direction or speed of fluid flow. This allows, for example, the LED UV emitters 120 to be turned on while fluid flows from inlet 112 to outlet 114 and turned off if flow stops or flow direction reverses. In still other embodiments, the sensor system 112 can include detectors to monitor levels of particulates or microorganisms.

The controller 130 can be used to set timing and duration of sterilization. In some embodiments, intensity can be dynamically changed in response to received sensor data from sensor system 122. In some embodiments, the controller 130 can provide visual status indication of detected sensor data or transmit that data to an external computing system such as smartphone

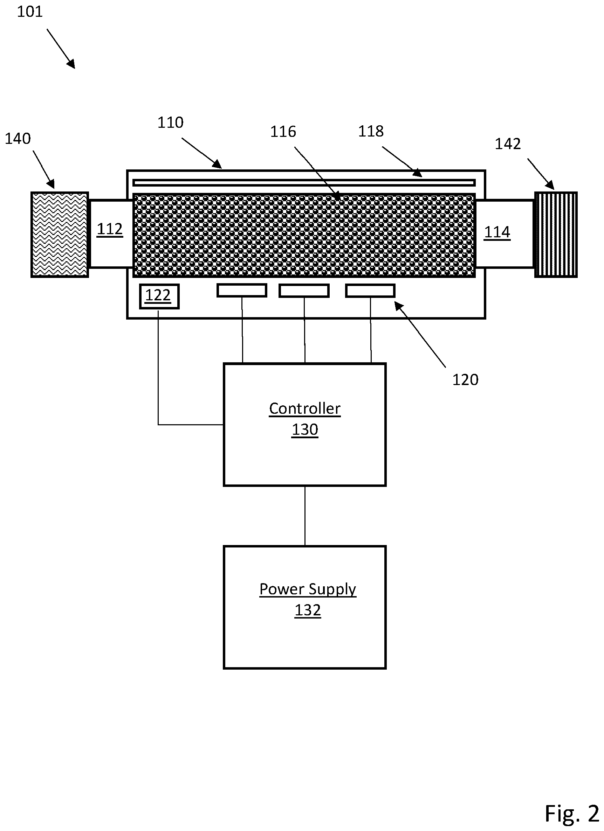

FIG. 2 is an illustration of a UV sterilization system 101 for air with optional prefilters 140 and post-filters 142. The prefilter 140 and post-filter 142 can include one or more of coarse and fine air filters, HEPA filters, chemical filters, or activated carbon filters. Both, or each alone of the filters 140 and 142 can be used. In this embodiment, size of the inlet and outlet are more closely matched to housing size, but operation of the system is otherwise as described with respect to FIG. 1.

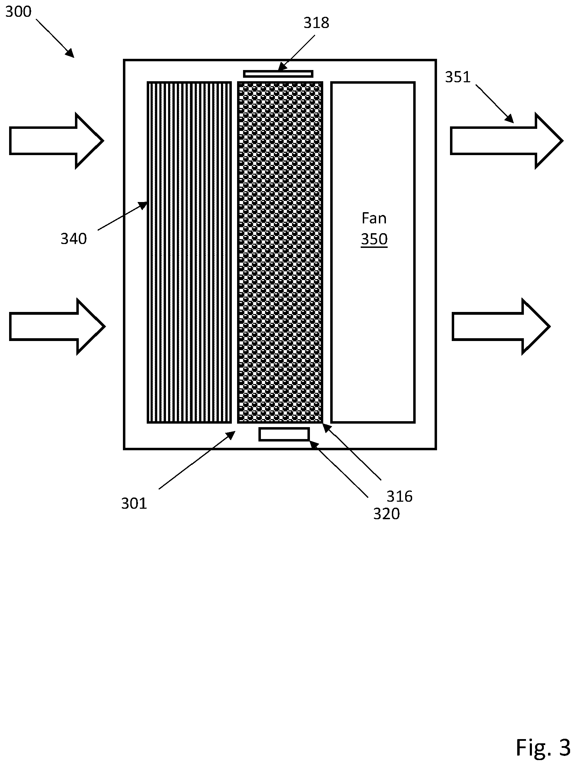

FIG. 3 is an illustration of a low power, small form factor air purification system 300 supporting UV sterilization. As illustrated, the system includes a pre-filter 340 and a UV sterilization system 301. A fan 350 pulls air through the pre-filter 340 and sterilization system 301 in a direction indicated by the arrows 351, releasing the purified air into a room or cabin of a vehicle. In this embodiment, the UV emitter 320 and UV reflector 318 direct light into a porous material 316 that has a short axis along the direction of air flow. Advantageously, such systems can be battery powered or powered by low voltage power supplies.

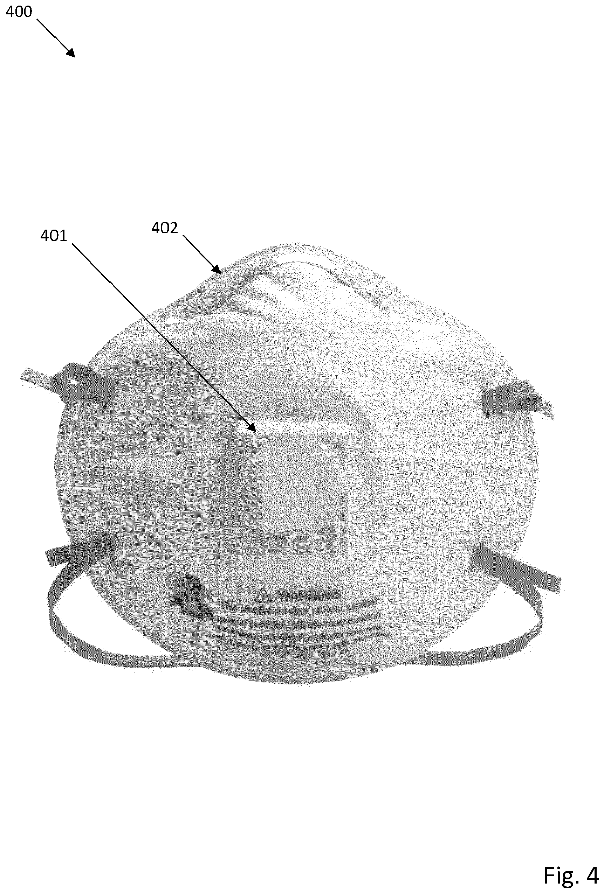

FIG. 4 is an illustration of a battery powered face mask 400 supporting UV sterilization with a battery powered sterilization unit 401. Advantageously, battery powered sterilization unit 401 can be attached to mask 402 as a permanent or removable module having a UV-absorbing material housing (e.g., ceramic, plastic, etc.) to prevent stray UV-light from exiting sterilization environment. The battery powered sterilization unit 401 can include a battery (power supply) and an air flow sensor that switches on during breath inhalation and off during breath exhalation. The battery powered sterilization unit 401 can also include a sterilization module (including a UV emitting LED, porous membrane, protective enclosure, gas flow environment), and a pre-filter to sterilization module (to remove large particles) similar to that discussed with respect to FIGS. 1-3. Advantageously, the pre-filter does not need to be N95 level (i.e., 300 nm pores), since bacteria and viruses can be destroyed by UV germicidal irradiation rather than trapped by physical particle filtration as in traditional N95 masks.

Having described the invention in detail, those skilled in the art will appreciate that, given the present disclosure, modifications may be made to the invention without departing from the spirit of the inventive concept described herein. Therefore, it is not intended that the scope of the invention be limited to the specific embodiments illustrated and described.

## Claims

1. A sterilization system, comprising:
a semiconductor LED emitter of UV radiation;
a fluid flow chamber positioned to receive UV radiation; and
a porous UV scattering medium positioned within the fluid flow chamber to scatter UV radiation while allowing fluid flow therethrough.

2. The LED of claim 1, wherein the UV radiation is UV-C radiation.

3. The LED of claim 1, wherein the fluid flow chamber further comprises a UV radiation reflective material.

4. The LED of claim 1, wherein the porous UV scattering medium further comprises porous quartz.

5. The LED of claim 1, wherein the porous UV scattering medium is sized to have pores that do not directly filter microorganisms.

6. The LED of claim 1, wherein the semiconductor LED emitter is battery powered.

7. The LED of claim 1, wherein the semiconductor LED emitter is powered by a low voltage electrical system.

8. The LED of claim 1, wherein the sterilization system is included in a portable system.

9. The LED of claim 1, wherein the sterilization system is included in a portable mask.

10. The LED of claim 1, wherein the sterilization system is included in an automotive air filtering system.

11. The LED of claim 1, wherein the sterilization system further comprises an air filtering system.

12. The LED of claim 1, further comprising an LED control system and a connected sensor, with semiconductor LED emitter operation varying according to sensed data received from the connected sensor.

13. A sterilization system, comprising:
a semiconductor LED emitter of UV radiation;
a fluid flow chamber positioned to receive UV radiation;
a porous UV scattering medium positioned within the fluid flow chamber to scatter UV radiation while allowing fluid flow therethrough.
an LED controller connected to a sensor system that monitors fluid flow through the fluid flow chamber, with the semiconductor LED emitter of UV radiation intensity controlled based at least in part on fluid flow conditions.

14. The LED of claim 13, wherein the semiconductor LED emitter of UV radiation emits sterilizing levels of UV radiation when fluid flows in a first direction and does not emit sterilizing levels of radiation when fluid flows in a second direction.

15. A sterilization method comprising:
Emitting sterilizing UV radiation with a semiconductor LED emitter;
positioning a fluid flow chamber to receive sterilizing UV radiation; and
allowing a porous UV scattering medium positioned within the fluid flow chamber to scatter UV radiation while allowing fluid flow therethrough.
